# EUROPÄISCHE PATENTANMELDUNG

(11) **EP 3 412 242 A1**
(43) Veröffentlichungstag der Anmeldung: **12.12.2018**
(21) Anmeldenummer: 17175269.4
(22) Anmeldetag: 09.06.2017
(51) Int. Cl.: A61B 90/00

(54) **AUSGABE VON POSITIONSINFORMATIONEN EINES MEDIZINTECHNISCHEN INSTRUMENTS**

(71) Anmelder: Siemens Healthcare GmbH, 91052 Erlangen (DE)
(72) Erfinder: Hofmann, Bernd, 91058 Erlangen (DE); Ullrich, Christian, 90587 Siegelsdorf (DE); Graupner, Harald, 91056 Erlangen (DE)

(57) **Zusammenfassung**

Die Erfindung befasst sich mit einem Verfahren zur Ausgabe von Positionsinformationen eines medizintechnischen Instruments (3), welches zumindest teilweise ein Körperareal (1) eines Patienten (2) penetriert. Ein Strukturbild (BS), ein Instrumentenbild (BIn) und ein Operationsszenenbild (BO) werden miteinander registriert, wobei sich aus der Registrierung ein registriertes Strukturbild (BrS), ein registriertes Instrumentenbild (BrI) und ein registriertes Operatiosszenenbild (BrO) ergeben. Es wird ein Überlagerungsbild (BU) mit einer VR- oder AR- Anzeigevorrichtung (17) angezeigt, welches zumindest Teile des registrierten Strukturbildes (BrS) des registrierten Operationsszenenbildes (BrO) - bzw. der realen Ansicht des entsprechenden Teils des realen Körperareals (1) - und des registrierten Instrumentenbilds (BrI) umfasst. Die Erfindung betrifft des Weiteren ein entsprechendes Bilderzeugungssystem.

## Beschreibung

Die Erfindung betrifft ein Verfahren sowie ein Bilderzeugungssystem zur Ausgabe von Positionsinformationen eines medizintechnischen Instruments, welches zumindest teilweise ein Körperareal eines Patienten penetriert, insbesondere zur Anzeige einer multisensorischen Darstellung dieses Körperareals.

Die Penetrationen eines menschlichen Körpers mit medizintechnischen Geräten, wie Endoskopen, beispielsweise eine Punktion von Hohlräumen innerhalb eines Schädels mit einem solchen Endoskop, ist eine gängige Methode zur Diagnostik und Therapie verschiedener Erkrankungen in der Medizin. Dabei wird das medizintechnische Instrument zunächst durch eine kleine Öffnung in den Körper eingeführt. Bei der Punktion des Ventrikelsystems wird beispielsweise das Instrument durch eine kleine Öffnung in die Schädelkalotte eingeführt, wobei in der Regel gesundes Hirngewebe mit dem Instrument durchstochen werden muss, um zum Zielort zu gelangen. Dabei muss das Instrument an lebensnotwendigen Strukturen vorbei dorthin manövriert werden. Aus diesen Gründen ist ein solcher Eingriff mit einer hohen Gefahr von Komplikationen verbunden, die durch eine Verletzung von Gewebe, z.B. Hirngewebe oder Gefäßen bedingt sein und möglicherweise mit inneren Blutungen oder mit neurologischen Ausfallserscheinungen einhergehen können.

Eine immer noch gängige Methode, insbesondere bei z.B. der Anlage einer sogenannten Ventrikeldrainage, ist die Blindpunktion. Das Erreichen des Zielortes erfolgt dabei ohne optische Kontrolle und basiert nur auf der Erfahrung des Operateurs. Daher sind oft mehrere Versuche notwendig, bis das Ziel erreicht wird. Dies geht naturgemäß mit einer erhöhten Gefahr für das Auftreten von Verletzungen und Komplikationen einher, in ungünstigen Fällen verbunden mit neurologischen Ausfällen oder einer verzögerten Rekonvaleszenz.

Eine Weiterentwicklung der Punktionstechnik beinhaltet die Nutzung von Navigationsverfahren (Neuronavigation), die erlaubt, den Weg des Instruments im Schädel zumindest indirekt auf einem Display zu verfolgen. Ein Nachteil einer solchen Darstellung ist die schwierige Führung des Instrumentes durch den Operateur, da das Display aus Sterilitätsgründen meist nicht in einer Linie mit der Führungsrichtung des Instrumentes aufgestellt werden kann und der Operateur somit entweder den Blick ständig zwischen Instrument und Display wenden oder eine ungünstige Haltung einnehmen muss. Dies alles führt dazu, dass auch hier - trotz Navigationsverfahren - das Ziel häufig nicht im ersten Punktionsversuch erreicht wird und sich somit die Komplikationsgefahr ebenfalls erhöht.

Es ist eine Aufgabe der vorliegenden Erfindung, ein sicheres und komfortableres Verfahren und ein entsprechendes Bilderzeugungssystem zur Darstellung eines Körperareals bereitzustellen, mit denen die oben beschriebenen Nachteile vermieden werden.

Diese Aufgabe wird durch ein Verfahren gemäß Patentanspruch 1 sowie durch ein Bilderzeugungssystem gemäß Patentanspruch 12 gelöst.

Das erfindungsgemäße Verfahren dient zur Ausgabe von Positionsinformationen von einem medizintechnischen Instrument, insbesondere einem Endoskop, wobei das medizintechnische Instrument zumindest teilweise ein Körperareal eines Patienten penetriert. "Patienten" im Rahmen dieser Erfindung können dabei im Grunde alle möglichen Lebewesen, Mensch oder Tier, aber auch unbelebte Objekte, z.B. Mumien sein.

Es handelt sich bei der Erfindung um ein automatisiertes Darstellungsverfahren von Bildern bzw. einem im Folgenden näher erläuterten Bilderzeugungssystem, welches solche Darstellungen ermöglicht. Auch wenn die Erfindung insbesondere einen Operateur im Rahmen eines Eingriffs unterstützen kann, betrifft sie nicht den Eingriff bzw. die Operationen an sich.

Das erfindungsgemäße Verfahren, welches insbesondere alternativ oder zusätzlich zur multisensorischen Darstellung eines Körperareals vor und/oder während der Penetration durch ein medizintechnisches Instrument geeignet ist, umfasst eine Anzahl von Schritten, die im Folgenden näher erklärt werden.

Zunächst erfolgt eine Bereitstellung, z.B. eine Erstellung, eines Strukturbildes und eines Instrumentenbildes. Eines dieser Bilder oder beide Bilder sind dazu vorzugsweise 3D-Darstellungen. Dies kann beispielsweise durch eine Bereitstellung von multidimensionalen Bilddaten geschehen, z.B. von CT-Bildern, die eine Aufnahme vom Inneren des betreffenden Körperareals enthalten. Der Begriff "multidimensional" meint dabei drei Raumdimensionen oder gegebenenfalls zusammen mit der Zeit vier Dimensionen, wobei die multidimensionalen Bilddaten auch in Form eines Stapels bzw. eine Serie von zweidimensionalen Bildern gegeben sein können. Das Körperareal kann dabei beispielsweise der Kopf oder der Brustkorb sein.

Das Strukturbild umfasst dabei innere Strukturen des betreffenden Körperareals. Diese Strukturen können durch eine vorher vorgenommene Aufnahme, z.B. oben genannte CT-Bilder, gewonnen werden, sie können aber auch direkt während des medizintechnischen Eingriffs gemacht werden. Insbesondere kann eine Aufbereitung der Bilddaten für das Strukturbild erfolgen, z.B. eine Entrauschung oder eine Erkennung bzw. Markierung von unterschiedlichen Strukturen in dem Bild.

Das Instrumentenbild umfasst mindestens den zur Operation verwendeten Teil des medizintechnischen Instruments bzw. dessen Bilddaten, die insbesondere in einer Datenbank als eine dreidimensionale Darstellung, z.B. eine CAD-Darstellung, abgespeichert sein können. Als Instrument werden alle Elemente angesehen, die für die Operation in das Körperareal eingebracht werden, wie z.B. Endoskope und Katheter. Des Weiteren sind z.B. auch Verbrauchsartikel wie beispielsweise Ventrikelkatheter medizintechnische Instrumente im Sinne der Erfindung.

Zusätzlich erfolgt eine Erstellung eines Operationsszenenbildes, beispielsweise durch eine direkte Aufnahme und/oder eine (Teil-)Rekonstruktion. Dieses Operationsszenenbild enthält Bilddaten umfassend zumindest eine Oberfläche des betreffenden Körperareals zusammen mit dem operierenden medizintechnischen Instrument, also bei einem Eingriff im Gehirn ein äußeres Bild zumindest eines Teils des Kopfes, an dem das Instrument in den Kopf eindringt. Es ist sinnvoll, dieses Operationsszenenbild während der Operation anzufertigen und ständig zu aktualisieren, um stets die aktuelle Operationsszene darzustellen. Das Operationsszenenbild zeigt die Oberfläche des Körperareals, wie z.B. den Kopf eines Patienten. In der Regel ist dies die Körperoberfläche, kann aber auch bei einem geöffneten Körperareal die Oberfläche von eigentlich innenliegenden Strukturen zeigen.

Für das Operationsszenenbild genügt im Grunde eine Kameraaufnahme des Körperareals zusammen mit Informationen über die reale relative Position und Orientierung des Körperareals und des medizintechnischen Instruments im Raum. Bevorzugt umfasst das Operationsszenenbild 3D-Daten des Patienten, insbesondere, wenn es von im Raum installierten Kameras aufgenommen wird, da je nach Blickwinkel des Operateurs eine individuell passende Ansicht erzeugt werden sollte.

Basierend auf den vorgenannten Bildern erfolgt zumindest bereichsweise eine automatische Registrierung des Operationszenenbilds, des Strukturbilds und des Instrumentenbilds aufeinander, wobei zumindest das Strukturbild und das entsprechende Körperareal in dem Operationsszenenbild sowie das Instrumentenbild mit dem medizintechnischen Instrument in dem Operationsszenenbild aufeinander registriert werden. Dabei ist es im Grunde unerheblich, welches dieser Bilder jeweils bei der Registrierung als Referenzbild dient, an welches das oder die anderen Bilder angepasst (auf dieses registriert) werden und welche die Objektbilder sind, die an das Referenzbild angepasst werden. Es kann auch jedes Bild im Rahmen der Registrierung an einen gemeinsamen Standard angepasst werden. Es bietet sich jedoch in vielen Fällen an, bei der Registrierung das Operationsszenenbild als Referenzbild zu verwenden, da dies die spätere Darstellung erleichtert.

Wichtig ist bei der Registrierung im Wesentlichen nur, dass das Strukturbild und das Instrumentenbild nach der Registrierung in das Operationsszenenbild mit der entsprechenden Größe, der entsprechenden Position und der entsprechenden Orientierung hineinpassen. Auch wenn bei der Registrierung eines der Bilder nicht verändert werden sollte, wird dennoch für ein besseres Verständnis nach der Registrierung von einem registrierten Strukturbild, einem registrierten Instrumentenbild und einem registrierten Operationsszenenbild gesprochen, die sich durch die Registrierung ergeben. Mit anderen Worten, das Strukturbild, das Instrumentenbild und das Operationsszenenbild werden zum registrierten Strukturbild, registrierten Instrumentenbild und registrierten Operationsszenenbild, indem sie den Registrierungsprozess durchlaufen haben.

Das registrierte Instrumentenbild entspricht dem Instrument des registrierten Operationsszenenbildes bzw. umgekehrt. Das registrierte Strukturszenenbild würde die innere Struktur des Körperareals korrekt wiedergeben, wenn dies in dem registrierten Operationsszenenbild transparent wäre.

Vor oder mit der Registrierung kann eine Erfassung der Position und Orientierung des entsprechend realen Körperareals relativ zum Operateur bzw. eine Erfassung der Position und Orientierung des realen medizintechnischen Instruments relativ zum Operateur erfolgen, z. B. mittels Auswertung des Operationsszenenbildes, sofern dies als 3D-Bild vorliegt oder zumindest aus einer Aufnahme zweier Kameras erstellt worden ist. Dies hat den Vorteil, dass eine einfachere und/oder genauere Registrierung des Strukturbilds bzw. des Instrumentenbildes erreicht werden kann.

In dem Falle, dass das Überlagerungsbild aus der Sicht des anwesenden Operateurs dargestellt werden soll, z.B. als Augmented Reality-Darstellung mit einer realen Operationsszene, ist es ggf. notwendig, das Operationsszenenbild entsprechend zu justieren. In dem Falle, dass das Operationsszenenbild, z.B. von Kameras an der Anzeigevorrichtung, direkt aus dem Blickwinkel des Operateurs aufgenommen worden ist, muss in der Regel kein zusätzlicher Aufwand betrieben werden. In dem Falle, in dem das Operationsszenenbild von fest oder beweglich installierten Raumkameras aufgenommen worden ist, sollte der Blickwinkel und die Position des Operateurs, bzw. dessen Anzeigevorrichtung, bestimmt werden, um das Operationsszenenbild entsprechend anzupassen. In Fällen, in denen das Operationsszenenbild sowohl von Aufnahmen von Kameras an der Anzeigevorrichtung am Operateur (z.B. in einer AR-brille installierten Kameras) als auch von Aufnahmen von Raumkameras gebildet wird, müssen diese unterschiedlichen Aufnahmen miteinander koordiniert bzw. registriert werden.

Nach der Registrierung erfolgt eine Anzeige eines Überlagerungsbilds mit einer VR- oder AR-Anzeigevorrichtung (VR: "Virtual Reality"; "virtuelle Realität" bzw. AR: "Augmented Reality"; "Erweiterte Realität").

Dieses Überlagerungsbild umfasst zumindest einen Teil des registrierten Strukturbilds in Form einer (registrierten) Überlagerung mit dem registrierten Operationsszenenbild oder der realen Ansicht des entsprechenden Teils des realen Körperareals und des registrierten Instrumentenbilds zumindest in demjenigen Bereich, in dem das medizintechnische Instrument in dem registrierten Operationsszenenbild von der Oberfläche des Körperareals verdeckt ist. In dem Überlagerungsbild befinden sich somit die entsprechenden Teile des registrierten Strukturbildes dort, wo sich auch in dem registrierten Operationsszenenbild die entsprechenden Strukturen finden ließen. Genauso befindet sich zumindest der Teil des registrierten Instrumentenbildes dort, wo das medizintechnische Instrument zu sehen wäre, wenn es sich nicht im Körperareal befinden würde. Durch das Überlagerungsbild wird also der Eindruck erzeugt, man könne in das Körperareal hineinschauen. Dies könnte man auch mit dem Begriff "Mixed Reality" (MR) bezeichnen.

Alternativ oder zusätzlich kann das Überlagerungsbild das Operationsszenenbild, das registrierte Operationsszenenbild oder die reale Ansicht des entsprechenden Teils des realen Körperareals zusammen mit einer Darstellung von Navigationsdaten für die Penetration umfassen. Das Überlagerungsbild kann also z. B. Markierungen für die Einstichstelle für die Penetration, den Weg zum Zielgebiet oder Richtungsinformationen enthalten.

In einem Beispiel, bei dem eine Ventrikelpunktierung stattfinden soll und das medizintechnische Instrument ein Endoskop und das Körperareal ein menschlicher Schädel ist, könnte das Überlagerungsbild als (registriertes) Strukturbild eine MRT- oder CT-Aufnahme des Gehirnbereichs mit dem Ventrikel umfassen, welche aufbereitet und mit der Ansicht des Schädels registriert worden ist. Dem Operateur würde sich durch die Anzeigevorrichtung eine Darstellung bieten, in der er stets die Spitze des Endoskops im Kopf sehen kann, da diese ihm durch das registrierte Instrumentenbild gezeigt wird. Zudem kann der Operateur stets das Gehirn und/oder die Ventrikel erkennen, da diese durch das registrierte Strukturbild dort abgebildet werden, wo sie sich bei der Sicht auf den Schädel des Patienten auch befinden würden.

Auch wenn vorzugsweise das Strukturbild - wie oben gesagt - ein 3D-Bild ist, kann es prinzipiell auch ein zweidimensionales Bild sein, wenn sichergestellt ist, dass es in der Ebene des (registrierten) Operationsszenenbilds liegt. Es ist bevorzugt, dass das registrierte Strukturbild ein zweidimensionales Bild ist, welches ggf. aus einem 3D-Bild erstellt worden ist, da dies den Rechenaufwand bei der Darstellung senkt. In der Regel werden wie erwähnt viele jeweils aktuelle Operationsszenenbilder nacheinander aufgenommen, bzw. das Verfahren vielfach durchlaufen, da sich der Blickwinkel des Operateurs bzw. die Position des medizintechnischen Instruments in der Praxis ständig ändert. Damit kann stets ein Überlagerungsbild erstellt werden, welches die aktuelle Situation bei der Operation als Basis hat.

Bevorzugt steht dem Operateur die Möglichkeit eines Zooms zur Verfügung. Dazu wird nach einem Befehl des Operateurs das dargestellte Überlagerungsbild mit all seinen Komponenten, also dem registrierten Operationsszenenbild, dem registrierten Strukturbild und dem registrierten Instrumentenbild vergrößert oder verkleinert.

Das erfindungsgemäße Bilderzeugungssystem zur Ausgabe von Positionsinformationen von einem medizintechnischen Instrument, welches zumindest teilweise ein Körperareal eines Patienten penetriert, ist insbesondere zur Ausführung des vorgenannten Verfahrens ausgelegt und umfasst zumindest die folgenden Komponenten:
- Eine Strukturbildschnittstelle, die ggf. auch als "Bilderstellungseinheit" bezeichnet werden kann, zur Bereitstellung eines Strukturbilds, welches innere Strukturen des Körperareals umfasst. Diese Strukturbildschnittstelle ist bevorzugt eine Schnittstelle, die Bilddaten einer medizintechnischen Vorrichtung, z. B. eines Computertomographen (CT), empfangen kann. Dazu ist sie insbesondere dazu ausgelegt, Daten nach dem DICOM-Standard (DICOM: "Digital Imaging and Communications in Medicine"; deutsch: "Digitale Bildgebung und -kommunikation in der Medizin") zu übernehmen. Vorzugsweise ist die Strukturbildschnittstelle auch dazu ausgelegt, die Bilddaten für das erfindungsgemäße Bilderzeugungssystem anzupassen, zum Beispiel, indem sie automatisch eine Konvertierung in das erforderliche Format vornimmt.
- eine Instrumentenbildschnittstelle zur Bereitstellung eines Instrumentenbildes umfassend mindestens einen Teil des medizintechnischen Instruments. Die Instrumentenbildschnittstelle ist bevorzugt dazu ausgelegt, auf eine Datenbank mit Instrumentenbildern zuzugreifen und Bilddaten, insbesondere 3D-Daten wie z. B. CAD-Daten, zu übernehmen und diese dem Bilderzeugungssystem zur Verfügung zu stellen.
- eine Aufnahmeeinheit, z. B. eine Kameraanordnung, zur Erstellung eines Operationsszenenbildes umfassend das betreffende Körperareal zusammen mit dem medizintechnischen Instrument. Die Aufnahmeeinheit ist dabei vorzugsweise zur Aufnahme von Bilddaten und besonders bevorzugt auch zur Bearbeitung derselben ausgelegt.
- eine Registrierungseinheit zur zumindest bereichsweisen Registrierung des Operationsszenenbilds, des Strukturbilds und des Instrumentenbilds aufeinander, wobei zumindest das Strukturbild und das entsprechende Körperareal in dem Operationsszenenbild sowie das Instrumentenbild mit dem medizintechnischen Instrument in dem Operationsszenenbild aufeinander registriert werden. Diese Registrierungseinheit ermöglicht dadurch die Erzeugung eines registrierten Strukturbildes und eines registrierten Operationsszenenbildes sowie eines registrierten Instrumentenbildes mittels Registrierung des Strukturbildes mit dem entsprechenden Körperareal in dem Operationsszenenbild und des Instrumentenbildes mit dem medizintechnischen Instrument in dem Operationsszenenbild. Bevorzugt erfolgt die Registrierung in einer Form, wie sie vorangehend im Zuge des Verfahrens bereits beschrieben wurde. Es kann dabei direkt mit den aufgenommenen Bildern gearbeitet werden oder mit berechneten Bildern, wie z. B. in dem Fall, dass Raumkameras das Operationsszenenbild aufnehmen und dieses so verändert werden soll, dass es den Blickwinkel des Operateurs auf die Szene korrekt wiedergibt.
- eine Schnittstelle für eine VR- oder AR-Anzeigevorrichtung, die zur Anzeige eines Überlagerungsbildes ausgestaltet ist. Je nach Ausführung des Bilderzeugungssystems kann auch die VR- oder AR-Anzeigevorrichtung von dem Bilderzeugungssystem umfasst sein. Die besagte Schnittstelle ist dabei so gestaltet, dass das Überlagerungsbild, welches für die VR- oder AR-Anzeigevorrichtung ausgegeben wird, zumindest einen Teil des registrierten Strukturbildes sowie das registrierte Instrumentenbild umfasst. Das registrierte Strukturbild liegt dabei in Form einer Überlagerung mit dem entsprechenden Körperareal in dem registrierten Operationsszenenbild oder der realen Ansicht des entsprechenden realen Körperareals vor. Das registrierte Instrumentenbild zeigt zumindest denjenigen Bereich, in dem das medizintechnische Instrument in dem registrierten Operationsszenenbild von der Oberfläche des Körperareals verdeckt ist.

Alternativ oder zusätzlich dazu umfasst das Überlagerungsbild, welches für die VR- oder AR-Anzeigevorrichtung ausgegeben wird, eine Überlagerung einer Darstellung von Navigationsdaten mit dem Operationsszenenbild, dem registrierten Operationsszenenbild oder mit der realen Ansicht des entsprechenden (realen) Körperareals.

Die vorliegende Erfindung ermöglicht also eine Ausgabe von Positionsinformationen über die Position des medizintechnischen Instruments in Form einer vorteilhaften Verbindung zwischen Bildgebung, automatisierter Bildverarbeitung und -erkennung sowie Augmented Reality bzw. Virtual Reality, so dass der Operateur quasi "sieht", wo er sich mit seinem Instrument in der Struktur des Körperareals gerade bewegt.

Ein Großteil der zuvor genannten Komponenten des Bilderzeugungssystems, insbesondere die Schnittstellen und/oder die Registrierungseinheit, können ganz oder teilweise in Form von Softwaremodulen in einem Prozessor einer entsprechenden Steuereinrichtung realisiert werden. Eine weitgehend softwaremäßige Realisierung hat den Vorteil, dass auch schon bisher verwendete Vorrichtungen auf einfache Weise durch ein Software-Update in das System integriert werden können, um auf die erfindungsgemäße Weise zu arbeiten. Insofern wird die Aufgabe auch durch ein entsprechendes Computerprogrammprodukt mit einem Computerprogramm gelöst, welches direkt in eine Speichereinrichtung einer Recheneinrichtung des Bilderzeugungssystems ladbar ist, mit Programmabschnitten, um alle Schritte des erfindungsgemäßen Verfahrens auszuführen, wenn das Programm in der Recheneinrichtung ausgeführt wird. Ein solches Computerprogrammprodukt kann neben dem Computerprogramm gegebenenfalls zusätzliche Bestandteile wie z. B. eine Dokumentation und/oder zusätzliche Komponenten, auch Hardware-Komponenten, wie z.B. Hardware-Schlüssel (Dongles etc.) zur Nutzung der Software, umfassen.

Zum Transport zur Recheneinrichtung und/oder zur Speicherung an oder in der Recheneinrichtung kann ein computerlesbares Medium, beispielsweise ein Memorystick, eine Festplatte oder ein sonstiger transportabler oder fest eingebauter Datenträger dienen, auf welchem die von einer Rechnereinheit der Recheneinrichtung einlesbaren und ausführbaren Programmabschnitte des Computerprogramms gespeichert sind. Die Rechnereinheit kann z. B. hierzu einen oder mehrere zusammenarbeitende Mikroprozessoren oder dergleichen aufweisen.

Weitere, besonders vorteilhafte Ausgestaltungen und Weiterbildungen der Erfindung ergeben sich aus den abhängigen Ansprüchen sowie der nachfolgenden Beschreibung. Dabei kann das erfindungsgemäße Verfahren bzw. das erfindungsgemäße Bilderzeugungssystem auch analog zu den abhängigen Verfahrensansprüchen oder Beschreibungsteilen weitergebildet sein. Insbesondere können auch einzelne Merkmale verschiedener Ausführungsbeispiele bzw. Varianten zu neuen Ausführungsbeispielen bzw. Varianten kombiniert werden.

Bevorzugt ist bezüglich des Überlagerungsbildes, dass diejenigen Teile des medizintechnischen Instruments, die in dem Operationsszenenbild sichtbar sind, als reales Abbild oder als Bilddaten des Operationsszenenbildes, also nicht als Teil des (registrierten) Instrumentenbildes in dem Überlagerungsbild dargestellt sind, vorzugsweise ab dem Übergang vom sichtbaren zum im Körperareal befindlichen Teil. Dadurch wird nur derjenige Teil des Instrumentenbildes, der im Operationsszenenbild nicht sichtbar ist, "virtuell" dargestellt, was die Darstellung für den Operateur verbessert. Durch die oben beschriebene erfindungsgemäße Registrierung ist dabei sichergestellt, dass das virtuelle Bild des Instruments exakt an der Position angezeigt wird, an der sich der entsprechende Teil des realen Instruments auch befindet.

Besonders bevorzugt ist, dass der registrierte Teil des Instrumentenbildes grafisch hervorgehoben ist, insbesondere die Bereiche, die im Operationsszenenbild nicht vom Instrument zu sehen sind, beispielsweise durch eine kräftigere oder abgewandelte Farbgebung. Dies hat den Vorteil, dass die verdeckten Bereiche des Instruments besser als solche wahrgenommen werden können.

Vorzugsweise werden in dem Überlagerungsbild bereits vom medizintechnischen Instrument durchquerte Bereiche des Körperareals, z. B. durchquertes Gewebe, in der Darstellung des registrierten Strukturbildes ausgeblendet oder ihre Transparenz erhöht. Dies hat den Vorteil, dass der Fokus des Operateurs auf die relevanten Bereiche und noch zu passierende Strukturen gelenkt wird. Technisch kann dies beispielsweise realisiert werden, indem in einer 3D-Darstellung des Strukturbilds all diejenigen Bereiche ausgeblendet werden, die außerhalb eines frei definierbaren Radius um die Zielstruktur liegen. Der Radius soll dabei den Abstand der Spitze des Instruments von der Zielstruktur nicht unterschreiten, d.h. beide Strukturen müssen in der Mixed Reality sichtbar sein. Dieser Abstand kann beispielsweise während der Registrierung ermittelt werden, da die Zielstruktur in dem Strukturbild bekannt ist und die Lage von (registriertem) Strukturbild und (registriertem) Instrumentenbild ebenfalls.

Besonders bevorzugt ist, dass bestimmte, definierte Strukturen im Strukturbild segmentiert werden und als eigenständige Bild- bzw. Daten-Objekte vorliegen. Vorzugsweise können beispielsweise speziell gekennzeichnete Strukturen nicht ausgeblendet werden oder Strukturen objektweise ausgeblendet werden. So können z. B. Adern weiterhin sichtbar bleiben, obwohl das medizintechnische Instrument sie bereits passiert hat, wohingegen anderes Körpergewebe ausgeblendet wird.

Bevorzugt wird zur Navigation basierend auf dem Strukturbild die Zielstruktur der Penetration ermittelt. Dies kann manuell geschehen, bevorzugt ist jedoch, dass eine automatische Erkennung der relevanten Zielstrukturen durchgeführt wird, z. B. mit Algorithmen zum maschinellen Lernen. Diese Zielstruktur und/oder der Weg zu ihr wird dann in dem Überlagerungsbild, insbesondere in dem registrierten Strukturbild, markiert bzw. für den Operateur in dem Überlagerungsbild räumlich simuliert.

In dem Fall, dass das medizintechnische Instrument eine Spitze aufweist, ist bevorzugt, dass die jeweils aktuelle Position der Spitze des medizintechnischen Instrumentes automatisch erkannt und in dem Überlagerungsbild markiert wird. Vorzugsweise geschieht dies im (registrierten) Instrumentenbild, z. B. unter Zuhilfenahme des 3D-Datensatzes und Navigationssoftware und/oder von Simulationsergebnissen.

Es ist ebenfalls bevorzugt, dass Hilfsinformationen, insbesondere Navigationsinformationen, z. B. Pfeile, Richtungsangaben, Entfernungen etc., in dem Überlagerungsbild eingeblendet werden, die während der Penetration das Erreichen der Zielstruktur erleichtern. Vorzugsweise zeigen diese Hilfsinformationen die jeweilige Richtung an, in der die Spitze des Instruments zur Zielstruktur geführt werden muss. Dies erleichtert dem Operateur während der Operation das Erreichen des Ziels. Die Hilfsinformationen können zweidimensionale Darstellungen sein, je nach Anwendung sind aber dreidimensionale Darstellungen bevorzugt.

Bevorzugt werden in dem registrierten Strukturbild bestimmte relevante, insbesondere sicherheitsrelevante, Strukturen des Körperareals gesondert markiert. Eine solche Markierung kann vorab manuell oder nach einer automatischen Erkennung mittels künstlicher Intelligenz auf automatische Weise erfolgen. Dabei ist bevorzugt, dass diese relevanten Strukturen farblich codiert von der Anzeigevorrichtung in dem Überlagerungsbild eingeblendet werden.

Alternativ oder ergänzend dazu ist bevorzugt, dass mögliche Kollisionen des medizintechnischen Instruments, insbesondere dessen Spitze, mit diesen relevanten Strukturen automatisch erkannt werden und ein Warnsignal ausgegeben wird. Ein solches Warnsignal kann akustischer, visueller und/oder haptischer Natur sein. Dies hilft, Komplikationen, z.B. Blutungen beim Verletzen von Gefäßen, zu vermeiden.

Bevorzugt wird die Zielstruktur oder ihre Position vorübergehend in dem Überlagerungsbild markiert, wenn diese das Sichtfeld der Anzeigevorrichtung verlassen hat und/oder wieder in das Sichtfeld kommt. Vorzugsweise kann die Zielstruktur in dem Fall, wenn sie das Sichtfeld temporär verlassen hat und danach wieder in das Bild kommt, vorübergehend gesondert in dem Überlagerungsbild markiert werden. Eine solche Markierung kann insbesondere farblich oder durch Vergrößerung erfolgen und hat den Vorteil, dass ein Wiederauffinden erleichtert wird. Vorzugsweise erfolgt diese vorübergehende Markierung kurzzeitig, wobei sie bevorzugt nicht länger als 5 Sekunden, insbesondere nicht länger als 1 Sekunde, bestehen bleibt. Dies beugt einer Ermüdung des Operateurs vor, dem das Auffinden kleiner Zielstrukturen erleichtert wird, und kann die Operationsdauer dadurch verkürzen. Bei optisch ähnlichen Strukturen hilft es außerdem, die relevante Zielstruktur sicher wiederzuerkennen, und erhöht damit ebenfalls die Sicherheit während der Operation. Alternativ oder zusätzlich kann auch eine Markierung der Zielstruktur, welche sich aktuell nicht mehr im Sichtfeld befindet, durch einen Hinweis, beispielsweise einen Pfeil oder dergleichen, erfolgen, der im Sichtfeld darauf hinweist, in welcher Richtung sich die Zielstruktur neben dem Sichtfeld befindet.

Bevorzugt umfasst das Bilderzeugungssystem Forced Feedback Generatoren (Kraftrückkopplungsgeneratoren), die eine haptische Rückkopplung ermöglichen und die insbesondere an oder in dem medizintechnischen Instrument angeordnet sind. Eine haptische Rückkopplung umfasst dabei beispielsweise eine Kraft, die der Bewegung des medizintechnischen Elements entgegenwirkt, einer Vibration, oder einer Erwärmung. Bevorzugte Forced Feedback Generatoren sind Vibratoren, Thermoelemente oder Kraftüberträger.

Bevorzugt ist, dass diese Forced Feedback Generatoren auf haptische Weise die Führung des medizintechnischen Instruments unterstützen und/oder bei einer drohenden Beschädigung von inneren Strukturen des Körperareals eine haptische Warnung aussenden. Dies geschieht bevorzugt durch Veränderung der Haptik des medizintechnischen Instruments. Insbesondere wird dabei der virtuelle Widerstand relevanter Strukturen sensorisch an den Operateur übertragen und dieser kann entscheiden, inwieweit er eine akzidentelle Verlagerung von z. B. Blutgefäßen in Kauf nimmt. Hilfreich bei der Generierung der Feedbackinformation kann dabei die Shear-Stress-Analyse dieser Strukturen sein, die über entsprechende Software erfolgt. Bevorzugt wird ein haptischer Eindruck mittels Vibrationselementen oder dergleichen erreicht.

Bevorzugt findet eine Überwachung einer Abweichung des räumlichen Verlaufs des im Körperareal befindlichen Teils des medizintechnischen Instruments von einem vorbestimmten Verlauf statt. Besonders bevorzugt wird bei Überschreiten einer maximal zulässigen Abweichung ein Warnsignal ausgegeben. Ein solches Warnsignal kann haptische und/oder akustische und/oder optische Komponenten aufweisen. Dies ist insbesondere dann von Vorteil, wenn sich der Operateur nur auf die Spitze des Instrumentes und das Zielgebiet fokussiert und dabei den Verlauf des von der Spitze entfernten Teils des Instruments akzidentell stark variiert. Diese Abweichungen vom originären Pfad können ebenfalls zu Verletzungen von relevanten Strukturen führen und sollten vermieden werden. Somit kann die Sicherheit bei der Operation ebenfalls erheblich erhöht werden.

Bevorzugt ist, dass mittels Steuerungsmechanismen Teile von virtuellen Abbildungsdaten und/oder zusätzliche Informationen in dem Überlagerungsbild ein- oder ausgeblendet werden und/ oder die Funktion von Geräten, insbesondere von medizintechnischen Geräten, gesteuert wird. Solche Steuerungsmechanismen können z. B. Fußschalter, bzw. sogenannte "intelligente" Steuermechanismen, wie z. B. Voicecontrol, und/oder Schaltflächen in dem Überlagerungsbild sein.

Beispielsweise könnte ein vom Endoskop erzeugtes "Endoskopbild" bzw. "Livebild" bei einer Videoendoskopie ein- oder ausgeblendet werden oder störende Informationen ausgeblendet werden. Letzteres ist hilfreich, um die zu behandelnden Zielstrukturen nicht von virtuellen Informationen "überblendet" zu sehen, was zu einer schlechteren Erkennbarkeit von kleinen Strukturen, die behandelt werden sollen, führen würde.

Bevorzugt ist, dass in dem Überlagerungsbild zusätzlich ein Endoskopbild enthalten ist, das insbesondere, z. B. wie vorgenannt erwähnt, gesteuert ein- und ausgeblendet werden kann. Dieses Endoskopbild kann vorzugsweise an einer Position im Überlagerungsbild bzw. im Raum innerhalb des Überlagerungsbilds virtuell fixiert werden. Dies kann in bevorzugter Weise an der Position der Endoskopspitze des registrierten Instrumentenbilds bzw. am Fokuspunkt der Endoskopoptik erfolgen. Daraus ergibt sich die vorteilhafte Möglichkeit, auch den endoskopisch durchgeführten Eingriff über die Anzeigevorrichtung, unabhängig von einem weiteren Monitor, durchzuführen. Dies erhöht die Sicherheit des Eingriffs, da der Operateur auch die extrakorporale "Achse" seines Instruments immer im Blickfeld hat, was eine stabilere manuelle Führung des Instruments nach sich zieht und somit die Komplikationsrate vermindert und den Eingriff beschleunigt. Bei dreidimensionaler Videoendoskopie erfolgt die Darstellung der körpereigenen Strukturen bevorzugt ebenfalls dreidimensional. Das Bildsignal des Endoskops wird dabei an geeigneten Schnittstellen, z. B. HDMI, abgegriffen und durch informationstechnische Algorithmen in das Überlagerungsbild überführt. Die Position der Endoskopkamera, bzw. der Kameraoptik und der Strukturen im Livebild kann anhand der errechneten Koordinate im Referenzsystem ermittelt werden.

In dem bevorzugten Fall, dass der Operateur die Möglichkeit eines Zooms hat, ist besonders bevorzugt, dass bei einer voreingestellten Vergrößerungsstufe des Überlagerungsbildes an der Position der Endoskopspitze das Endoskopbild eingeblendet wird, vorzugsweise in einer Größe, in der die im Endoskopbild dargestellten Strukturen der Größe und Position der entsprechenden Strukturen des registrierten Strukturbildes haben. Besonders bevorzugt ist, dass das Endoskopbild mit dem Strukturbild registriert wird.

Bevorzugt umfasst das Operationsszenenbild Informationen zur Position und Orientierung des Körperareals und des medizintechnischen Instruments im Raum. Es wird vorzugsweise aus Bildern erstellt, die mittels Kameras aufgenommen werden, wobei die betreffenden Kameras bevorzugt in der Anzeigevorrichtung integriert sind und/oder im Raum positioniert sind. Eine Kombination beider Kameraanordnungen ist dabei besonders bevorzugt.

Die Kameras können dabei fest in dem Raum verbaut sein, aber auch mobil sein, wie z.B. an der Anzeigeeinrichtung des Operateurs befestigt sein, oder auf andere Weise im Raum beweglich sein. Es ist bei Raumkameras, die nicht automatisch mit dem Kopf des Operateurs bewegt werden, von Vorteil, wenn sie ihre Position nach einer initialen Einstellung im Raum nicht mehr ändern. Wenn dennoch eine Positionsänderung im Raum erfolgt, ist es von Vorteil, wenn der Raum im Bereich der aufgenommenen Operationsszene fixe Strukturen aufweist, z.B. einen Marker am OP-Tisch, damit eine Zuordnung der Operationsszene im Raum möglich ist, insbesondere relativ zum Operateur und dessen Blickrichtung. Diesbezüglich ist bevorzugt, dass die Anzeigevorrichtung des Operateurs so gestaltet ist, dass ihre Position und Orientierung im Raum auf den Operationsszenenbildern ermittelt werden kann.

Bevorzugt werden zur Erstellung des Operationsszenenbilds, insbesondere zur Ermittlung der Position und Orientierung des Körperareals und des Instruments im Raum, zusätzlich auch Informationen aus anderen bildgebenden Systemen oder 3D-Sensoren verwendet. Solche Systeme oder Sensoren können beispielsweise Beschleunigungsmesser, Sender oder Empfänger für Ortsinformationen sein.

Bei Durchführung des Verfahrens wird also wie oben beschrieben die Zielstruktur, z. B. einer Punktion, bzw. die Oberflächenkontur des Patienten vorzugsweise zunächst in einem Schnittbilddatensatz identifiziert. Zur Anwendung kommt dabei insbesondere eine automatisierte Identifikation, bei der der Operateur lediglich in einem oder in wenigen Schnittbildern die Zielstruktur markiert. Die Oberfläche des Patienten wird dabei bevorzugt automatisch erkannt.

Vorzugsweise erfolgt dann die Registrierung von virtuellem Ziel und Oberfläche des Patienten (auch als "Koregistrierung" bezeichnet). Hierbei erfolgt die Registrierung des Patienten insbesondere über Kameras, die im Raum aufgestellt sind, oder in optimaler Weise mit Hilfe von Kameras, die in der AR-Brille integriert sind. Letzteres bietet den Vorteil, dass sich der Aufwand der Reprojektion minimiert, weil nicht mehr die Sicht des Benutzers aus dem Bezugssystem zusätzlich extrapoliert werden muss.

Zur Erhöhung der Detaillierung und Präzision können auch Informationen aus anderen bildgebenden Systemen oder 3D-Sensoren verwendet werden. Bevorzugte bildgebenden Systeme oder 3D-Sensoren könnten bereits in der Kollisionsdetektion innerhalb des bildgebenden Systems zum Einsatz kommen bzw. es könnten deren Koordinatensysteme oder dreidimensionale Bilddaten verwendet werden. Diese Informationen können ebenfalls zu einer Registrierung verwendet werden. Dieses Verfahren bietet den Vorteil, dass sich der Aufwand der Reprojektion minimiert, weil nicht mehr die Sicht des Benutzers aus dem Bezugssystem zusätzlich extrapoliert werden muss. Hierzu werden die aufgenommenen Bilder genutzt, um per stereoskopischer Reprojektion auf die physischen Dimensionen des Patienten und des ihn umgebenden Raumes zu schließen.

Somit kann eine zusätzlich angebrachte Referenzmarkierung die Orientierung auch bei einem steril abgedeckten Patienten ermöglichen, bei Registrierung in Koordinatensystemen verschiedener Systeme kann ggf. diese Referenzmarkierung auch entfallen.

Insgesamt wird somit die virtuelle Oberfläche des Patienten mit der realen automatisch registriert und damit auch die Läsion virtuell "im Körper" des Patienten abgebildet. Diese kann dann dem Operateur im Brillendisplay dargestellt werden.

Es wird wie oben beschrieben auch das medizintechnische Instrument, das zur Operation - z. B. zur Punktion - genutzt wird, im virtuellen Raum registriert und in ein virtuelles Abbild überführt, welches mit dem realen Instrument, zumindest dem nicht sichtbaren Teil, überlagert wird. Bei Eindringen des Instruments in den Körper des Patienten kommt dann nicht mehr nur das reale Bild des Instruments zur Darstellung, sondern das virtuelle Bild wird partiell, d. h. zumindest ab dem Übergang vom sichtbaren zum im Körper befindlichen Teil, für den Operateur abgebildet. Somit ist es dem Operateur möglich, in bequemer Haltung ständig den Patienten im Blick zu haben und das Instrument in optimaler Arbeitsposition zielgerichtet und nur mit kleinen Korrekturen anhand des virtuellen Bildes zum Ziel vorzuschieben. Es steht zu erwarten, dass somit ein erfolgreicher Eingriff, z. B. eine Punktion, insbesondere von kleinen Strukturen im ersten Versuch erfolgt und das Komplikationsrisiko deutlich vermindert wird.

Aus diesen Vorteilen ergibt sich neben der Verringerung der Komplikationsquoten eine Reduktion der OP-Zeit, was insgesamt nicht nur für den Pateinten gut ist, sondern auch eine Entlastung der Gesundheitssysteme erreicht.

Darüber hinaus kann unter Umständen in einigen Fällen sogar die Durchführung einer postoperativen Kontrollbildgebung unterbleiben, was zu einer Reduktion der Strahlenbelastung des Patienten und einer Kostenreduktion führen kann.

Die Erfindung wird im Folgenden unter Hinweis auf die beigefügten Figuren anhand von Ausführungsbeispielen noch einmal näher erläutert. Dabei sind in den verschiedenen Figuren gleiche Komponenten mit identischen Bezugsziffern versehen. Die Figuren sind in der Regel nicht maßstäblich. Es zeigen:
- Figur 1: eine Darstellung einer Kopfoperation nach dem derzeitigen Stand der Technik,
- Figur 2: eine Darstellung einer Kopfoperation mit Unterstützung durch eine bevorzugte Ausführungsform eines erfindungsgemäßen Bilderzeugungssystems,
- Figur 3: eine Darstellung einer bevorzugten Ausführungsform eines erfindungsgemäßen Bilderzeugungssystems zusammen mit Bereitstellungs- und Darstellungskomponenten,
- Figur 4: eine Darstellung eines bevorzugten Überlagerungsbildes,
- Figur 5: eine Darstellung des Ablaufs einer bevorzugten Ausführungsform eines erfindungsgemäßen Verfahrens,
- Figur 6: eine Darstellung eines Flussdiagramms zum Ablauf einer Beispieloperation.

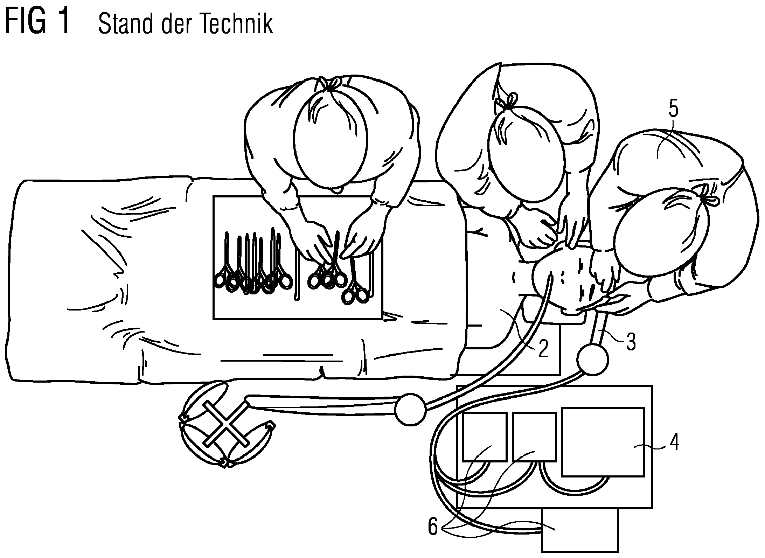

Figur 1 zeigt eine Darstellung einer Kopfoperation, z. B. der Punktierung eines Ventrikels nach dem derzeitigen Stand der Technik. Ein Patient 2 liegt auf einer Liege und wird von einem Operateur 5 mit einem medizintechnischen Instrument 3, z. B. einem Endoskop, operiert. Auf einem Display 4 kann er ein Videobild sehen, welches von einer Kamera aufgenommen wird, die sich in der Spitze des medizintechnischen Instruments 3 befindet. Ein Instrumentenhilfssystem 6 unterstützt die Funktion/Navigation des medizintechnischen Instruments 3.

Nachteil bei dieser Anordnung ist, dass der Operateur 5 ständig seinen Kopf drehen muss, um das Display 4 zu betrachten. Während dieser Bewegung kann sich die Lage des medizintechnischen Instruments 3 im Kopf des Patienten 2 ein wenig ändern, so dass der Operateur 5 ein Bild sehen könnte, was während seiner Operationstätigkeit so nicht erschienen ist, oder dass nach Betrachten eines Bildes und Bewegen des Kopfes die Spitze des medizintechnischen Instruments 3 sich nicht mehr in der Position befindet, in der das betrachtete Bild aufgenommen worden ist. Weiterhin kann es durch unbeabsichtigte Bewegungen zu einer Verletzung relevanter Strukturen kommen.

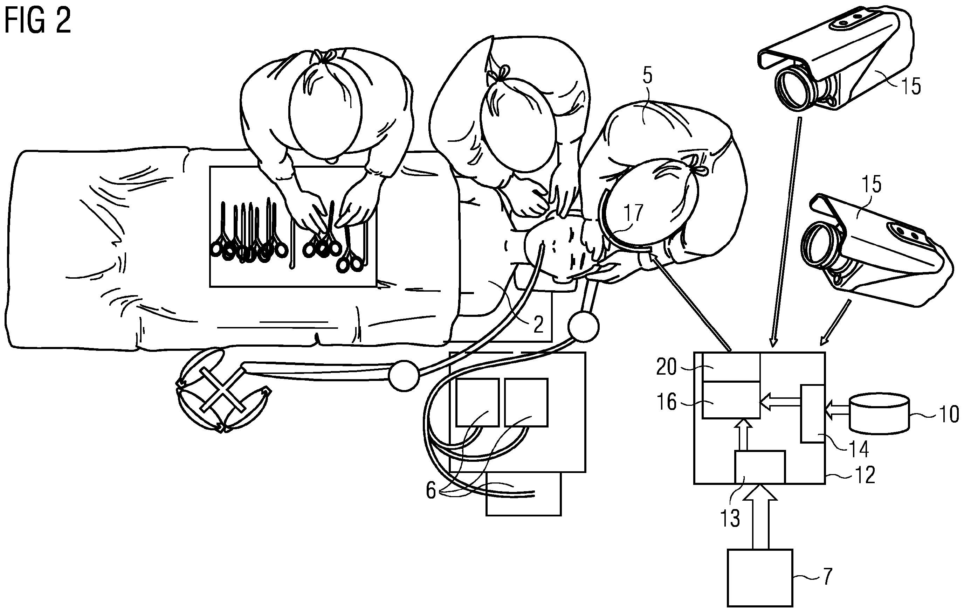

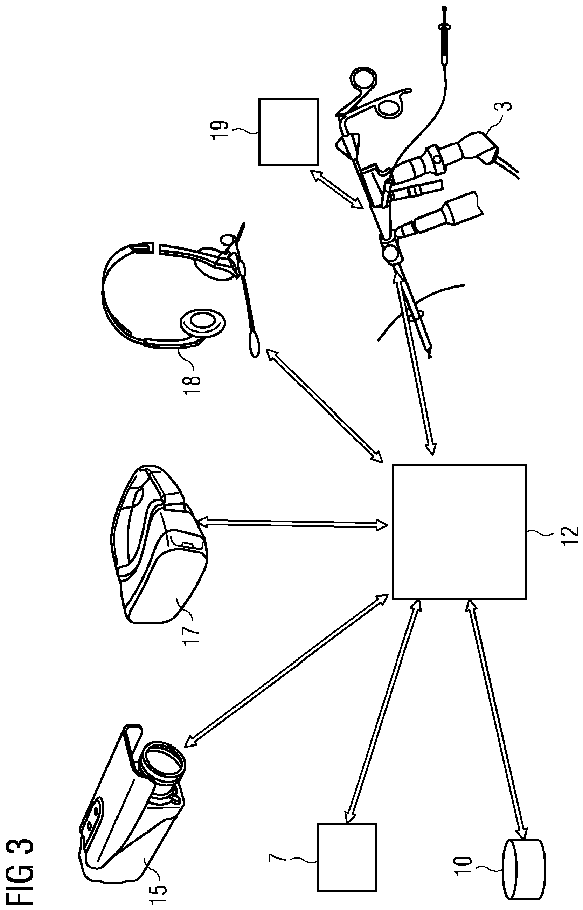

Figur 2 zeigt eine Darstellung einer Kopfoperation mit Unterstützung durch eine bevorzugte Ausführungsform eines erfindungsgemäßen Bilderzeugungssystems 12. Auch hier wird ein Patient 2 von einem Operateur 5 mittels eines medizintechnischen Instruments 3 am Kopf operiert. Im Unterschied zu Figur 1 muss der Operateur 5 jedoch nicht mehr auf ein Display 4 schauen, sondern trägt eine AR-Brille 17 als AR-Anzeigevorrichtung 17 eines Überlagerungsbilds BU, wie es z. B. in Figur 3 dargestellt ist.

Dieses Überlagerungsbild UB wird von dem Bilderzeugungssystem 12 erzeugt und mittels der Schnittstelle 20 zu der AR-Brille 17 gesendet. Dies kann kabelgebunden erfolgen, aber auch drahtlos, z. B. mittels WLAN oder Bluetooth.

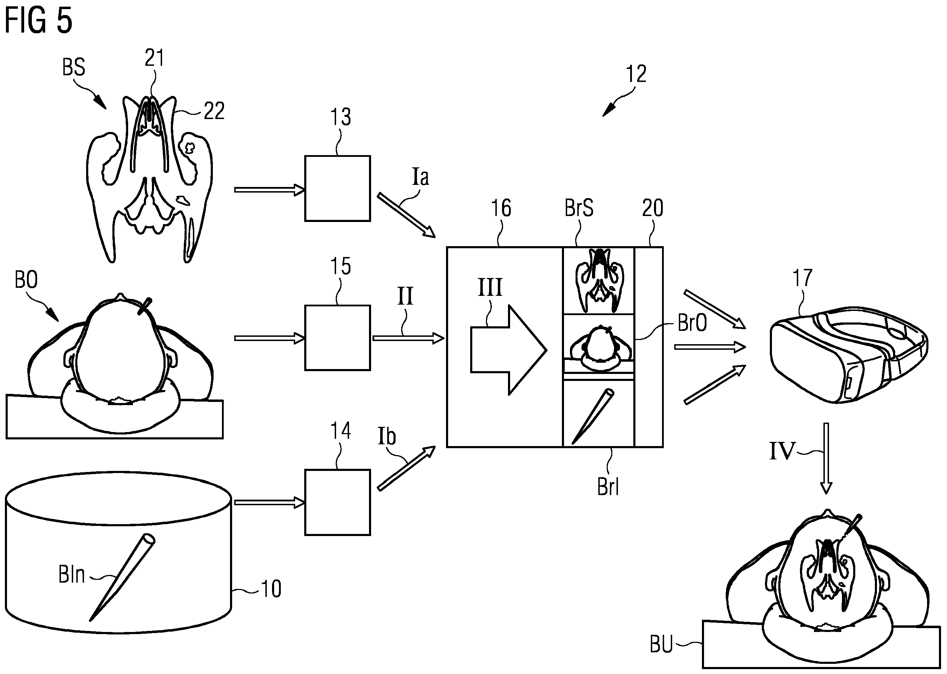

Das Bilderzeugungssystem 12 umfasst eine Strukturbildschnittstelle 13, welche von einem Computertomograph 7 oder einem sonstigen medizintechnischen Bildgebungssystem, wie einem Magnetresonanztomographen, erzeugte Bilddaten- vom Inneren des Kopfes 1 des Patienten 2 empfangen kann. Diese Bilddaten wurden im Vorfeld angefertigt und umfassen ein Strukturbild BS, wie es z. B. in Figur 5 dargestellt ist. In dem Strukturbild BS sind die zu punktierenden Ventrikel 22 als Zielstruktur und zudem Blutgefäße 21 zu erkennen, die sicherheitsrelevante Strukturen darstellen. Vor der Operation hat das Bilderzeugungssystem 12 das Strukturbild BS empfangen.

Das Bilderzeugungssystem 12 umfasst des Weiteren eine Instrumentenbildschnittstelle 14, welche CAD-Bilddaten von einer Datenbank 10 empfangen kann. Diese CAD-Bilddaten wurden im Vorfeld in einem CAD-Programm gemäß den Abmessungen des realen medizintechnischen Instruments 3 angefertigt und stellen das Instrumentenbild BIn dar, wie es z. B. ebenfalls in Figur 5 dargestellt ist. In dem Instrumentenbild BIn ist insbesondere die Spitze des Endoskops dargestellt, die in den Kopf des Patienten geschoben werden kann. Vor der Operation hat das Bilderzeugungssystem 12 das Instrumentenbild empfangen.

Zwei Kameras 15 dienen als Aufnahmeeinheiten für das Operationsszenenbild BO, wie es z. B. ebenfalls in Figur 5 dargestellt ist, und stellen dem Bilderzeugungssystem 12 ihre aufgenommenen Bilddaten zur Verfügung. Die Kameras 15 sind hier im Raum dargestellt, sie können aber auch Kameras 15 sein, die in die AR-Brille 17 integriert sind. Auch können mehr als zwei Kameras genutzt werden. Alternativ oder zusätzlich könnte auch eine Stereokamera oder dergleichen zum Einsatz kommen.

Eine Registrierungseinheit 16 führt eine Bildregistrierung des Strukturbilds BS, des Instrumentenbilds BIn und des Operationsszenenbilds BO aufeinander durch und erzeugt ein registriertes Strukturbild BrS, ein registriertes Instrumentenbild BrI und ein registriertes Operationsszenenbild BrO. Dieser Vorgang wird unten im Rahmen der Figur 5 genauer beschrieben.

Verschiedene Komponenten, die mit dem Bilderzeugungssystem 12 interagieren können bzw. Teil des Bilderzeugungssystems 12 sein können, sind in Figur 3 noch einmal schematisch aufgezeigt. Doppelpfeile zwischen den Komponenten sollen den Datenfluss darstellen, der vom Bilderzeugungssystem 12 zu den Komponenten und von den Komponenten zum Bilderzeugungssystem 12 verlaufen kann. Die Komponenten sind die vorgenannte Datenbank 10, in der sich die Informationen zum Instrumentenbild BIn befinden, der Computertomograph 7, der die Bilddaten für das Strukturbild BS liefert, eine Kamera 15, die zur Erstellung des Operationsszenenbilds BO gebraucht wird, die AR-Brille 17, welche das Überlagerungsbild anzeigt, Steuerungsmechanismen 18, wie beispielsweise das Headset, mittels dessen eine Steuerung des Bilderzeugungssystems 12 mit Wortbefehlen möglich ist, und ein medizintechnisches Instrument 3, welches mit Forced Feedback Generatoren 19, wie z. B. Vibratoren, ausgestattet ist, die durch das Bilderzeugungssystem 12 über das medizintechnische Instrument 3 zu Warnungen oder einem haptischen Feedback angesteuert werden können.

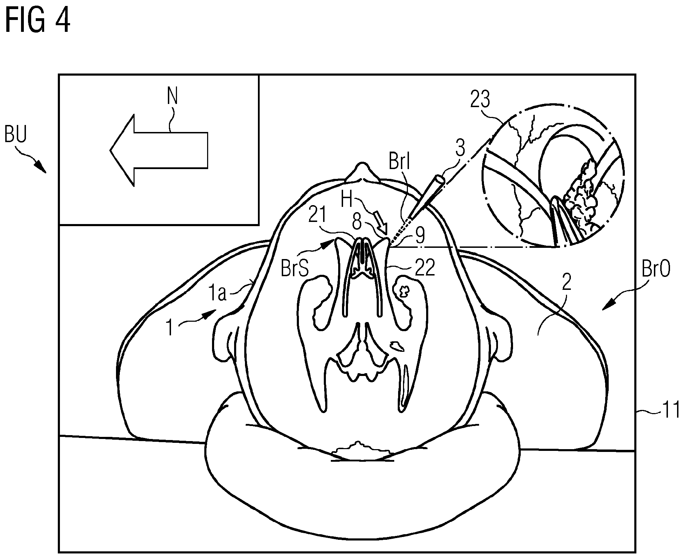

Figur 4 zeigt eine Darstellung eines bevorzugten Überlagerungsbilds BU, wie es beispielsweise mit dem vorangehend beschriebenen Bilderzeugungssystem 12 erzeugt und mittels der AR-Brille 17 in ihrem Sichtfeld 11 angezeigt werden könnte.

Basis für das Überlagerungsbild BU ist das registrierte Operationsszenenbild BrO, welches den Patienten 2 aus der Sicht des Operateurs 5 in Figur 2 zeigt und welches auch nur teilweise dargestellt werden könnte. Anstelle des registrierten Operationsszenenbildes BrO könnte auch die reale Ansicht des Patienten 2 gezeigt sein, die von den übrigen Bildern im Rahmen der Augmented Reality überlagert wird. Es wäre auch möglich, dass im Überlagerungsbild die Operationsszene teilweise real und teilweise in Form des Operationsszenenbilds dargestellt wird. Der Operateur 5 kann sich bei einer bevorzugten Ausführungsform auch im Operationssaal bewegen, wobei das Überlagerungsbild BU bevorzugt so gestaltet ist, dass es stets das Gesichtsfeld des Operateurs 5 wiedergibt.

In der Realität sieht der Operateur 5 lediglich den Patienten 2, das Körperareal 1, hier den Kopf 1, und zwar lediglich dessen Oberfläche 1a, und das medizintechnische Element 3, jedoch nur die Teile, die nicht in den Kopf 1 des Patienten 2 eingedrungen sind. Andere Elemente, die in Figur 4 eingezeichnet sind, werden durch das erfindungsgemäße Verfahren ergänzt. So sieht der Operateur 5 nun auch das registrierte Instrumentenbild BrI des im Kopf befindlichen Teils des medizintechnischen Elements 3, insbesondere dessen Spitze 9, die zusätzlich markiert sein könnte, z. B. mittels Hilfsinformationen H, wie einem kleinen Pfeil. Die Hilfsinformationen könnten aber auch zur Kenntlichmachung der Zielstruktur 8 dienen, die ebenfalls besonders markiert sein könnte.

Die gesamte innere Struktur des Kopfes 1 des Patienten 2 kann als registriertes Strukturbild BrS in dem Überlagerungsbild BU angezeigt werden. Im vorliegenden Fall hat die Spitze 9 des medizintechnischen Elements 3 bereits fast den Ventrikel 22 erreicht, und die äußeren Teile des Gehirns wurden für eine bessere Sicht auf das Ziel ausgeblendet. Einzig der automatisch als Objekt bzw. Zielstruktur 22 erkannte Ventrikel 22 und eine sicherheitsrelevante Struktur 21 aus Adern bleibt im Überlagerungsbild BU eingeblendet, so dass der Operateur sich optimal zurechtfinden kann.

Auch wenn in der Darstellung die Spitze 9 des medizintechnischen Instruments 3 anscheinend die Zielstruktur 8 erreicht hat, erkennt das Bilderzeugungssystem, dass sich die Spitze 9 etwas zu weit rechts befindet. Daher werden in der linken oberen Ecke Navigationsinformationen N eingeblendet, die anzeigen, dass der Operateur 5 die Spitze 9 noch etwas nach links bewegen muss.

In der Figur ist auch der bevorzugte Fall dargestellt, in der dem Operateur ein Livebild 23 eines Endoskops 3, also ein Endoskopbild 23, zur Verfügung steht. In dem dargestellten Fall wird es in der rechten oberen Ecke als Vergrößerung des Bereichs der Spitze 9 gezeigt. Es ist aber auch wie oben beschrieben möglich, das Endoskopbild 23 direkt im Bereich der Spitze 9 anzuzeigen, insbesondere im Falle eines Zooms in das Überlagerungsbild hinein.

Figur 5 zeigt eine Darstellung des Ablaufs einer bevorzugten Ausführungsform eines erfindungsgemäßen Verfahrens. Auf der linken Seite sind schematisch das Strukturbild BS, das Instrumentenbild BIn und das Operationsszenenbild BO gezeigt. Das Strukturbild BS wird im Schritt Ia dem Bilderzeugungssystem 12 über seine Strukturbildschnittstelle 13 bereitgestellt, das Instrumentenbild BIn von der Datenbank 10 durch die Instrumentenbildschnittstelle 14 und das Operationsszenenbild BO durch die Aufnahmeeinheit 15. Die Bereitstellung für das Bilderzeugungssystem 12 bedeutet hier, dass alle drei Bilder in einem Registrierungsschritt III von der Registrierungseinheit 16 verarbeitet werden, welche ein registriertes Strukturbild BrS, ein registriertes Instrumentenbild BrI und ein registriertes Operationsszenenbild BrO erzeugt. In der Praxis wird oftmals das Operationsszenenbild BO als Referenzbild für die Registrierung genutzt werden. In dem Fall, dass die Aufnahmeeinheit 15 in der AR-Brille angeordnet ist, muss das Operationsszenenbild noch nicht einmal verändert werden. Trotzdem wird aus Gründen der Übersichtlichkeit auch ein nicht verändertes Bild als "registriert" bezeichnet, wenn der Registrierungsschritt III durchlaufen worden ist.

Die registrierten Bilder werden wie beschrieben zu einem Überlagerungsbild BU überlagert und über die Schnittstelle 20 an die AR-Brille 17 gesendet, wobei der Überlagerungsschritt IV wie dargestellt in der AR-Brille 17 erfolgen kann oder aber im Bilderzeugungssystem, wobei das Überlagerungsbild BU direkt an die AR-Brille gesendet wird.

Das hier am Beispiel der Anlage einer Ventrikeldrainage dargestellte Verfahren kann analog für jedes Instrument, das in den Schädel oder andere Körperhohlräume oder Organe (z. B. bei Biopsien) eingeführt wird, angewandt werden.

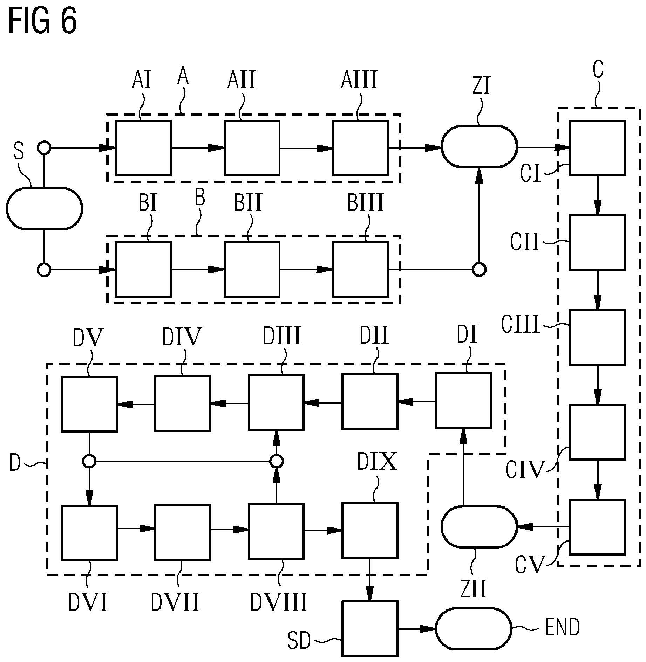

Figur 6 zeigt eine Darstellung eines Flussdiagramms zum Ablauf einer Beispieloperation, wobei noch einmal darauf hingewiesen wird, dass die Operation insgesamt nicht Teil der Erfindung ist, sondern nur die bildgebenden Aspekte. Die Figur dient insofern nur zur Einordnung der erfindungsgemäßen Schritte in einem Kontext einer bevorzugten Nutzung der Erfindung zur Unterstützung.

Nach einer Startprozedur START werden zwei Routinen A und B durchlaufen, deren Ergebnisse in Routine C zusammengefasst und verarbeitet werden. Diese übergibt ihre Ergebnisse an Routine D, welche den Kern des Verfahrens bildet. Nach Beendigung der Routine D erfolgt ein Shutdown SD der Systeme und das Ende des Ablaufs END.

In Routine A, die der Vorbereitung des Strukturbildes BS dient, wird zunächst in Schritt AI der Patient in einem Computertomographen, einem Magnetresonanztomographen oder einem anderen bildgebenden medizintechnischen Gerät durchleuchtet und im Schritt AII eine 3D-Rekonstruktion der inneren Strukturen, insbesondere zusammen mit der äußeren Kontour, erzeugt. Die Einbeziehung der äußeren Kontur hat den Vorteil, dass später eine Zuordnung zu dem Operationsszenenbild einfacher wird. In einem letzten Schritt AIII werden die aufgenommenen Daten abgespeichert und Routine C zur Verfügung gestellt.

In Routine B, die der Vorbereitung der Aufnahme des Operationsszenenbilds BO beziehungsweise einer Reihe von vielen Operationsszenenbildern BO dient, werden zunächst in Schritt BI Kameras im Operationsraum oder an der AR-Brille bereitgestellt. Danach folgt im Schritt BII eine Kalibrierung der Bilddaten bezüglich des Raums, so dass später eine Ansicht des Patienten 2 aus jedem erforderlichen Blickwinkel erzeugt werden kann. Die Kalibrationsmatrix wird in Schritt BIII dem weiteren Verfahren zur Verfügung gestellt, damit später das registrierte Operationsszenenbild BrO einfach aus dem Operationsszenenbild BO erzeugt werden kann.

Im Zwischenschritt ZI werden zunächst die Daten der Routinen A und B an die Routine C übergeben, die der direkten Vorbereitung dient. Dort werden in Schritt CI die Kameras gestartet, in Schritt CII die Kalibrationen der Kameras überprüft und in Schritt CIII Links zwischen den Datenflüssen zu den verschiedenen Ein- und Ausgabegeräten hergestellt. Danach werden in Schritt CIV eine Berechnung der Transformationsmatrix zwischen zwei/oder n 3-Dimensionalen Abbildungen der Kamerasysteme anhand von Ankerpunkten durchgeführt und es wird zuletzt in Schritt CV die Beziehungen zwischen Vektoren anhand von einfachen Tests getestet, beispielsweise anhand von Normvektoren und deren Vergleich im jeweils anderen System.

Im Zwischenschritt ZII werden die Daten der Routine C an die Routine D übergeben.

Während die Routinen A bis C lediglich der Vorbereitung der Bilder und der Systeme dienen, findet in Routine D der eigentliche Vorgang, z. B. die Bildgebung und Registrierung, statt. Der Patient 2 wird in Schritt DI im Operationssaal positioniert und in Schritt DII wird seine Position kalibriert bzw. das System auf seine Position kalibriert. Nun wird in Schritten DIII bis DVIII eine Schleife durchlaufen, um die Verfolgung des medizinischen Instruments 3 im Patienten 2 so oft wie möglich zu validieren. Zunächst wird in Schritt DIII der Patient 2 lokalisiert, danach in Schritt DIV die Lokalisation des Körperareals, also dem Ort, an dem operiert werden soll, im Raum gesucht, dann in Schritt DV die Konturen des Strukturbildes mit den realen Konturen registriert, z.B. mit der Kopfhaut bei einer Kopfoperation, das medizinische Instrument 3 in Schritt DVI lokalisiert und in Schritt DVII das Instrumentenbild BIn in das Überlagerungsbild eingefügt. In einem nächsten Schritt DVIII erfolgt die Aktion des Operateurs 5. Zuletzt wird nach erfolgter Operation in Schritt DIX der Patient 2 wieder aus dem Operationssaal verbracht.

In erster Linie ist von der Erfindung eine Reduktion der Komplikationsraten zu erwarten, da durch die verbesserte Art der Darstellung selbst kleine Strukturen im ersten Operationsversuch erreicht werden können. Dazu trägt auch die optimale Arbeitshaltung des Chirurgen bei, der mittels der Erfindung sozusagen "unter Sicht" die Strukturen erreichen kann.

Ein weiterer Vorteil ergibt sich aus der automatisierten Erstellung des Überlagerungsbilds und der optischen Registrierung von Patient, Instrument und Strukturbild über Kameras, insbesondere in der VR- bzw. AR-Anzeigevorrichtung. Gerade bei einer Aufnahme des Operationsszenenbilds mittels Kameras an der Anzeigevorrichtung steht zu erwarten, dass dies sehr viel schneller möglich ist als die Durchführung der Registrierung, bei der zunächst Kameras im Raum aufgestellt und auf den Patienten ausgerichtet werden müssen. Auch wird eine höhere Genauigkeit des Überlagerungsbilds erwartet, da das Sichtfeld des Operateurs und damit der Kameras im Normalfall immer auf die in der "Region of Interest" gelegenen Referenzpunkte ausgerichtet ist und somit keine Interferenz mit anderen Gegenständen, die in das Blickfeld der Kameras geschoben werden, auftreten kann.

Es wird abschließend noch einmal darauf hingewiesen, dass es sich bei den vorhergehend detailliert beschriebenen Verfahren sowie bei dem dargestellten System 12 lediglich um Ausführungsbeispiele handelt, welche vom Fachmann in verschiedenster Weise modifiziert werden können, ohne den Bereich der Erfindung zu verlassen. Weiterhin schließt die Verwendung der unbestimmten Artikel "ein" bzw. "eine" nicht aus, dass die betreffenden Merkmale auch mehrfach vorhanden sein können. Ebenso schließen die Begriff "Einheit" und "Modul" nicht aus, dass die betreffenden Komponenten aus mehreren zusammenwirkenden Teil-Komponenten bestehen, die gegebenenfalls auch räumlich verteilt sein können.

## Patentansprüche

1. Verfahren zur Ausgabe von Positionsinformationen eines medizintechnischen Instruments (3), welches zumindest teilweise ein Körperareal (1) eines Patienten (2) penetriert, umfassend die folgenden Schritte:
a) Bereitstellung
- eines Strukturbilds (BS) umfassend innere Strukturen des Körperareals (1) und
- eines Instrumentenbilds (BIn) umfassend mindestens den zur Operation verwendeten Teil des medizintechnischen Instruments (3),
b) Erstellung eines Operationsszenenbilds (BO) umfassend zumindest eine Oberfläche (1a) des betreffenden Körperareals (1) zusammen mit dem operierenden medizintechnischen Instrument (3),
c) zumindest bereichsweise Registrierung des Operationsszenenbilds (BO), des Strukturbilds (BS) und des Instrumentenbilds (BIn) aufeinander, wobei zumindest das Strukturbild (BS) und das entsprechende Körperareal (1) in dem Operationsszenenbild (BO) sowie das Instrumentenbild (BIn) mit dem medizintechnischen Instrument (3) in dem Operationsszenenbild (BO) aufeinander registriert werden,
d) Anzeige eines Überlagerungsbilds (BU) mit einer VR- oder AR-Anzeigevorrichtung (17)
- wobei das Überlagerungsbild (BU) zumindest einen Teil des registrierten Strukturbilds (BrS) in Form einer Überlagerung mit dem registrierten Operationsszenenbild (BrO) oder der realen Ansicht des entsprechenden Teils des realen Körperareals (1) umfasst und zumindest einen Teil des registrierten Instrumentenbilds (BrI) in demjenigen Bereich umfasst, in dem das medizintechnische Instrument (3) in dem registrierten Operationsszenenbild (BrO) von der Oberfläche (1a) des Körperareals (1) verdeckt ist,
und/oder
- wobei das Überlagerungsbild (BU) das Operationsszenenbild (BO), das registrierte Operationsszenenbild (BrO) oder die reale Ansicht des entsprechenden Teils des realen Körperareals (1) zusammen mit einer Darstellung von Navigationsdaten (N) für die Penetration umfasst.

2. Verfahren nach Anspruch 1, wobei die Teile des medizintechnischen Instruments (3), die in dem Operationsszenenbild (BO) sichtbar sind, als reales Abbild oder als Bilddaten des Operationsszenenbildes (BO) in dem Überlagerungsbild (BU) dargestellt sind.

3. Verfahren nach einem der vorangehenden Ansprüche, wobei bereits vom medizintechnischen Instrument (3) durchquerte Bereiche des Körperareals (1) in der Darstellung des registrierten Strukturbildes (BrS) ausgeblendet werden oder ihre Transparenz erhöht wird.

4. Verfahren nach einem der vorangehenden Ansprüche, wobei zur Navigation basierend auf dem Strukturbild (BS) die Zielstruktur (8) der Penetration ermittelt und die Zielstruktur (8) und/oder ein Weg zu der Zielstruktur (8) in dem Überlagerungsbild (BU), insbesondere in dem registrierten Strukturbild (BrS), markiert wird.

5. Verfahren nach einem der vorangehenden Ansprüche,
wobei das medizintechnische Instrument (3) eine Spitze (9) aufweist und die jeweils aktuelle Position der Spitze (9) des medizintechnischen Instrumentes automatisch in dem Überlagerungsbild (BU) markiert wird
und/oder
wobei Hilfsinformationen (H) in dem Überlagerungsbild (BU) eingeblendet werden, die während der Penetration das Erreichen der Zielstruktur (8) erleichtern.

6. Verfahren nach einem der vorangehenden Ansprüche, wobei in dem registrierten Strukturbild (BrS) bestimmte relevante Strukturen (21) des Körperareals (1) markiert werden, wobei bevorzugt
- diese relevanten Strukturen (21) farblich codiert von der Anzeigevorrichtung (17) in dem Überlagerungsbild eingeblendet werden, und/oder
- mögliche Kollisionen des medizintechnischen Instruments (3) mit diesen relevanten Strukturen (21) automatisch erkannt werden und insbesondere ein Warnsignal ausgegeben wird.

7. Verfahren nach einem der Ansprüche 4 bis 6, wobei die Zielstruktur (8) oder ihre Position vorübergehend in dem Überlagerungsbild (BU) markiert wird, wenn diese das Sichtfeld (11) der Anzeigevorrichtung (17) verlassen hat und/oder wieder in das Sichtfeld kommt.

8. Verfahren nach einem der vorangehenden Ansprüche, wobei mittels Forced Feedback Generatoren (19) auf haptische Weise die Führung des medizintechnischen Instruments (3) unterstützt wird und/oder bei einer drohenden Beschädigung von bestimmten inneren Strukturen des Körperareals (1) eine haptische Warnung ausgesendet wird.

9. Verfahren nach einem der vorangehenden Ansprüche, wobei eine Abweichung des räumlichen Verlaufs des im Körperareal (1) befindlichen Teils des medizintechnischen Instruments (3) von einem vorbestimmten Verlauf überwacht wird und bei Überschreiten einer maximal zulässigen Abweichung ein Warnsignal ausgegeben wird.

10. Verfahren nach einem der vorangehenden Ansprüche, wobei mittels vorzugsweise intelligenter Steuerungsmechanismen (18) Teile von virtuellen Abbildungsdaten und/oder zusätzliche Informationen in dem Überlagerungsbild (BU) ein- oder ausgeblendet werden und/oder die Funktion von Geräten gesteuert werden.

11. Verfahren nach einem der vorangehenden Ansprüche, wobei das Operationsszenenbild (BO) aus Bildern erstellt wird, die mittels Kameras (15) aufgenommen werden, wobei die betreffenden Kameras (15) bevorzugt in der Anzeigevorrichtung (17) integriert sind und/oder im Raum positioniert sind.

12. Bilderzeugungssystem (12) zur Ausgabe von Positionsinformationen eines medizintechnischen Instruments (3), welches zumindest teilweise ein Körperareal (1) eines Patienten (2) penetriert, umfassend
- eine Strukturbildschnittstelle (13) zur Bereitstellung eines Strukturbildes (BS) umfassend innere Strukturen des Körperareals (1),
- eine Instrumentenbildschnittstelle (14) zur Bereitstellung eines Instrumentenbildes (BIn) umfassend mindestens einen Teil des medizintechnischen Instruments (3),
- eine Aufnahmeeinheit (15) zur Erstellung eines Operationsszenenbildes (BO) umfassend zumindest eine Oberfläche (1a) des betreffenden Körperareals (1) zusammen mit dem medizintechnischen Instrument (3),
- eine Registrierungseinheit (16) zur zumindest bereichsweisen Registrierung des Operationsszenenbilds (BO), des Strukturbilds (BS) und des Instrumentenbilds (BIn) aufeinander, wobei zumindest das Strukturbild (BS) und das entsprechende Körperareal (1) in dem Operationsszenenbild (BO) sowie das Instrumentenbild (BIn) mit dem medizintechnischen Instrument (3) in dem Operationsszenenbild (BO) aufeinander registriert werden,
- eine Schnittstelle (20) für eine VR- oder AR-Anzeigevorrichtung (17), die ausgestaltet ist zur Anzeige eines Überlagerungsbildes (BU),
i) wobei das Überlagerungsbild (BU) zumindest einen Teil des registrierten Strukturbilds (BrS) in Form einer Überlagerung mit dem registrierten Operationsszenenbild (BrO) oder der realen Ansicht des entsprechenden Teils des realen Körperareals (1) umfasst und zumindest einen Teil des registrierten Instrumentenbilds (BrI) in demjenigen Bereich umfasst, in dem das medizintechnische Instrument (3) in dem registrierten Operationsszenenbild (BrO) von der Oberfläche (1a) des Körperareals (1) verdeckt ist
und/oder
ii) wobei das Überlagerungsbild (BU) das Operationsszenenbild (BO), das registrierte Operationsszenenbild (BrO) oder die reale Ansicht des entsprechenden Teils des realen Körperareals (1) zusammen mit einer Darstellung von Navigationsdaten (N) für die Penetration umfasst.

13. Bilderzeugungssystem nach Anspruch 12, welches Forced Feedback Generatoren (19) umfasst, welche ausgebildet sind, um auf haptische Weise die Führung des medizintechnischen Instruments (3) zu unterstützen und/oder bei einer drohenden Beschädigung von inneren Strukturen des Körperareals (1) eine haptische Warnung auszusenden, wobei die Forced Feedback Generatoren (19) insbesondere an oder in dem medizintechnischen Instrument (3) angeordnet sind.

14. Computerprogrammprodukt mit einem Computerprogramm, welches direkt in eine Speichereinrichtung einer Darstellungsvorrichtung (17) eines Darstellungssystems ladbar ist, mit Programmabschnitten, um alle Schritte des Verfahrens nach einem der Ansprüche 1 bis 11 auszuführen, wenn das Computerprogramm in der Darstellungsvorrichtung (17) des Darstellungssystems ausgeführt wird.

15. Computerlesbares Medium, auf welchem von einer Rechnereinheit einlesbare und ausführbare Programmabschnitte gespeichert sind, um alle Schritte des Verfahrens nach einem der Ansprüche 1 bis 11 auszuführen, wenn die Programmabschnitte von der Rechnereinheit ausgeführt werden.
